# EUROPÄISCHE PATENTSCHRIFT

(11) **EP 0 521 826 B1**
(45) Veröffentlichungstag und Bekanntmachung des Hinweises auf die Patenterteilung: **07.06.1995**
(21) Anmeldenummer: 92810489.2
(22) Anmeldetag: 25.06.1992
(51) Int. Cl.: C09D 5/24, H01B 1/12

(54) **Verfahren zur Herstellung von elektrisch leitenden Schichten**
Process for the production of electroconductive layers
Procédé pour la fabrication de couches électroconductrices

(30) Priorität: 02.07.1991 CH 1951/91
(43) Veröffentlichungstag der Anmeldung: 07.01.1993
(73) Patentinhaber: CIBA-GEIGY AG, 4002 Basel (CH)
(72) Erfinder: Hofherr, Walther, Dr., W-7815 Kirchzarten (DE); Minder, Ernst, CH-4450 Sissach (CH); Hilti, Bruno, CH-4054 Basel (CH); Ansermet, Jean-Philippe Dr., CH-1110 Morges (CH)

(56) Entgegenhaltungen:
- EP-A- 0 285 564
- EP-A- 0 362 141

## Beschreibung

Die Erfindung betrifft ein Verfahren zur Herstellung von elektrisch leitenden Schichten aus gegebenenfalls mit einem Kunststoff umhüllten Netzwerken von Kristallnadeln elektrisch leitender Radikalkationensalze von Tetrathio-, Tetraseleno- oder Tetratellurotetracenen und Halogenen oder Metallhalogeniden, bei dem man Suspensionen der Radikalkationensalze in einem inerten Lösungsmittel, in dem gegebenenfalls ein Kunststoff gelöst ist, oder bei dem eine gegebenenfalls einen Kunststoff enthaltende Lösung dieser Tetracene und Metallhalogenide oder eine Lösung dieser Tetracene und einer unter Wärmeeinwirkung mit diesen Tetracenen ein Halogenid (Radikalkationensalz) bildenden organischen Verbindung mittels Düsen auf die Oberfläche eines Trägermaterials sprüht und danach das Lösungsmittel verdampft.

In der EP-A-0 285 564 werden Trägermaterialien mit elektrisch leitenden Polymerschichten beschrieben, die ein Nadelnetzwerk von Radikalkationensalzen aus Tetrathio-, Tetraseleno- oder Tetratellurotetracenen und Metallsalzen oder Halogenen in der Polymermatrix enthalten. In der EP-A-0 362 141 werden Trägermaterialien offenbart, die nur mit Nadelnetzwerken solcher Radikalkationensalze beschichtet sind. In der EP-A-0 362 142 werden Trägermaterialien beschrieben, bei denen diese Nadelnetzwerke in halogenhaltigen Polymeren eingebettet sind. In der EP-A-0 362 143 sind Trägermaterialien beschrieben, bei denen diese Nadelnetzwerke in halogenhaltigen und photoempfindlichen Polymeren eingebettet sind. Die Herstellung der Schichten mit den Nadelnetzwerken erfolgt durch das Aufbringen von gebenenfalls ein Polymer enthaltenden Lösungen dieser Tetracene und unter Wärmeeinwirkung mit diesen Tetracenen ein Halogenid (Radikalkationensalz) bildenden organischen halogenhaltigen Verbindungen oder Polymeren auf ein Trägermaterial und ein darauf folgendes Abdampfen des Lösungsmittels.

Für das Aufbringen von Schichten werden übliche Verfahren vorgeschlagen, zum Beispiel Streichen, Rakeln und Giessen. Bei diesen Verfahren wird im allgemeinen eine Ausrichtung von Kristallnadeln in Richtung des Aufbringens beobachtet und ferner werden relativ weitmaschige Netzwerke aus Kristallnadeln gebildet, die aber eine hohe elektrische Leitfähigkeit aufweisen. Die Oberflächenentladung wird jedoch als zu langsam angesehen.

Mit diesen Verfahren können auch keine unregelmässigen Körper beschichtet und keine sehr dünnen Schichten erzielt werden. Die Viskosität der bei diesen Verfahren eingesetzten Lösungen kann nur in einem engen Bereich variieren.

Es wurde nun überraschend gefunden, dass man sehr feinmaschige, homogene und isotrope Netzwerke aus Kristallnadeln der erwähnten Radikalkationensalze herstellen kann, wenn man die Beschichtung auf das Trägermaterial aufsprüht. Hierbei können die Viskositäten der Lösungen in einem weiten Bereich variiert und die Länge der Kristallnadeln sowie die Maschenweite der Netzwerke durch die Grösse der Spraytropfen gezielt beeinflusst werden. Die elektrischen Leitfähigkeiten der Beschichtungen sind mit denen nach den erwähnten anderen Verfahren hergestellten Schichten vergleichbar, während die Oberflächenentladung überraschend besser ist. Ferner werden auch hervorragende Haftungen auf den Trägermaterialien, besonders auf Glas erzielt.

Gegenstand der Erfindung ist somit ein Verfahren zur Herstellung einer elektrisch leitenden Schicht auf einem Trägermaterial, bei dem die elektrisch leitende Schicht aus einem Netzwerk von Kristallnadeln elektrisch leitender Radikalkationensalze der Formel (I) 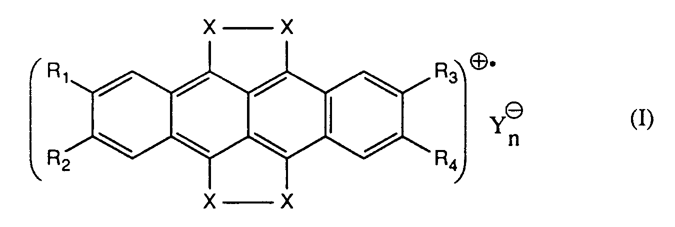
besteht und das Netzwerk in einer Polymermatrix eingebettet sein kann, worin X für S, Se oder Te steht, n eine Zahl von 0,3 bis 0,9 bedeutet, R₁ für H, F, Cl oder CH₃ und R₂, R₃ und R₄ für H stehen, oder R₁, R₂, R₃ und R₄ jeweils F oder CH₃ darstellen, oder R₁ und R₂ jeweils F, Cl oder CH₃ und R₃ und R₄ jeweils H bedeuten, oder R₁ und R₃ oder R₁ und R₄ jeweils F und R₂ und R₄ oder R₂ und R₃ jeweils H darstellen, und Y Cl, Br, I oder CuCl₂ bedeutet, durch Beschichten mindestens einer Oberfläche des Trägermaterials mit a) einer Suspension von Kristallnadeln der Radikalkationensalze der Formel I in einem inerten Lösungsmittel, das zusätzlich einen thermoplastischen Kunststoff oder mindestens eine Ausgangsverbindung für einen duroplastischen Kunststoff gelöst enthalten kann, oder b) einer Lösung von (b1) einer Verbindung der Formel II 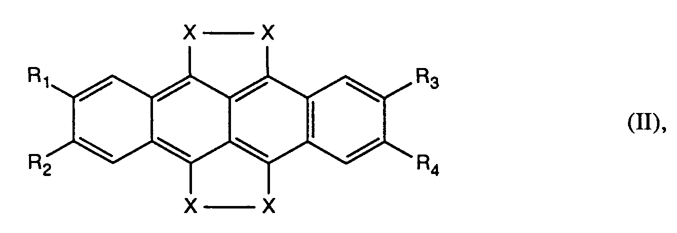
worin R₁, R₂, R₃, R₄ und X die zuvor angegebenen Bedeutungen haben, (b2) einer monomeren, oligomeren oder polymeren und Cl-, Br- oder I-haltigen organischen Verbindung, die unter Wärmeeinwirkung mit einer Verbindung der Formel II eine Verbindung der Formel I bildet, oder wasserfreiem CuCl₂, einem CuCl₂-Aquokomplex oder einem CuCl₂-Lösungsmittelkomplex, und (b3) gegebenenfalls einem thermoplastischen Kunststoff oder mindestens einer Ausgangsverbindung für einen duroplastischen Kunststoff, in einem inerten Lösungsmittel und anschliessendem Verdampfen des Lösungsmittels, das dadurch gekennzeichnet ist, dass die Schicht mittels Sprühen durch Düsen auf das Trägermaterial aufgebracht wird.

Die Düsen können aus unterschiedlichem Material bestehen, zum Beispiel Metallen, Metallegierungen, Glas und Kunststoffen. Bevorzugt bestehen die Düsen aus Glas oder Edelstahl.

Der Durchmesser der Düsenöffnungen kann zum Beispiel 0,1 bis 10 mm, bevorzugt 0,1 bis 5mm betragen.

Der für das Sprühen notwendige Druck wird zweckmässig mit Treibgasen erzeugt. Als Treibgase verwendet man im allgemeinen Edelgase, Stickstoff oder Luft, bevorzugt Argon oder Stickstoff.

Der Druck der Treibgase kann zum Beispiel 10⁴ bis 10⁶ Pa, bevorzugt 2·10⁴ bis 5·10⁵ Pa betragen.

Die Temperatur der Düsenköpfe kann zum Beispiel von Raumtemperatur bis 250 °C, bevorzugt von 50 bis 200 °C betragen.

Der Abstand der Düsenköpfe zum Trägermaterial beträgt im allgemeinen von 1 bis 100 cm, bevorzugt 5 bis 50 cm.

Es besteht die Möglichkeit, mehr als einen Düsenkopf, zum Beispiel 2 bis 10 Düsenköpfe gleichzeitig zu verwenden, wobei gleiche oder verschiedene Lösungen zur Beschichtung eingesetzt werden können.

Als Trägermaterialien kommen zum Beispiel Glas, Metalle, Metallegierungen, Halbleiter, Kunststoffe, mineralische und keramische Werkstoffe, Holz und Papier in Frage. Die Oberflächen von Kunststoffen können mit den verwendeten Lösungsmitteln quellen, so dass reine Netzwerke aus Kristallnadeln etwas in diese Oberflächen eindringen können. Die Trägermaterialien können beliebige äussere Formen aufweisen; es kann sich zum Beispiel um Formkörper, Fäden, Fasern, Gewebe, Stangen, Rohre, Bänder, Folien, Platten, Walzen oder Gehäuse handeln.

Die Konzentration der Verbindungen der Formel I und II kann 0,1 bis 20 Gew.-%, bevorzugt 0,1 bis 10 Gew.-% und besonders bevorzugt 0,1 bis 5 Gew.-% betragen, bezogen auf die Suspension beziehungsweise die Lösung.

Bei der Mitverwendung von Polymeren oder Ausgangsverbindungen für Polymere kann deren Konzentration in der Lösung bis zu 60 Gew.-%, bevorzugt 0,1 bis 50 Gew.-% und insbesondere 0,2 bis 30 Gew.-% betragen.

In einer Ausführungsform des erfindungsgemässen Verfahrens kann das Aufbringen der Schicht nach elektrostatischen Methoden erfolgen.

In einer bevorzugten Ausführungsform wird das Trägermaterial vorerwärmt, zum Beispiel auf bis zu 250 °C, bevorzugt auf 50 bis 200 °C.

Die Radikalkationensalze der Formel I, worin Y für Cl, Br oder I stehen, sind bekannt und zum Beispiel in den eingangs erwähnten europäischen Patentanmeldungen beschrieben. Die Radikalkationensalze der Formel I, worin Y für CuCl₂ steht, sind neu und können in an sich bekannter Weise durch die Umsetzung von wasserfreiem CuCl₂, CuCl₂-Aquokomplexen oder CuCl₂-Lösungsmittelkomplexen mit Tetrathio-, Tetraseleno- beziehungsweise Tetratellurotetracen in einem organischen Lösungsmittel hergestellt werden.

Lösungsmittelkomplexe von Kupferdichlorid und polaren aprotischen oder polaren protischen Lösungsmitteln sind in grosser Anzahl bekannt. Es können monomere, dimere und polymere Komplexe verwendet werden. Geeignete Lösungsmittel sind hauptsächlich solche mit Heteroatomen wie zum Beispiel Sauerstoff, Schwefel, Phosphor und Stickstoff. Einige Beispiele sind Ether (Diethylether, Dibutylether, Ethylenglykoldimethyl- oder -diethylether), Ester und Lactone (Essigsäureethylester, γ-Butyrolacton), Sulfone (Dimethylsulfon, Tetramethylensulfon) und Amine (Pyridin, α-Pyridon, α-Methylpyridin, Ethylendiamin, N,N-Dimethylethylendiamin, 1-(β-Aminoethyl)pyridin, 1-(β-Methylaminoethyl)-pyridin.

Die Reaktion wird in Gegenwart eines inerten Lösungsmittels durchgeführt. Geeignete Lösungsmittel, die alleine oder als Mischung von Lösungsmitteln eingesetzt werden können, sind zum Beispiel aliphatische und aromatische Kohlenwasserstoffe, wie z.B. Pentan, Hexan, Cyclohexan, Methylcyclohexan, Benzol, Nitrobenzol, Toluol, Xylol und Biphenyl; Alkohole wie z.B. Methanol, Ethanol, Propanol und Butanol; Ether wie z.B. Diethylether, Diethylenglykoldimethylether, Ethylenglykoldimethylether, Diphenylether, Tetrahydrofuran und Dioxan; Halogenkohlenwasserstoffe wie z.B. Methylenchlorid, Chloroform, 1,1,2,2-Tetrachlorethan und Chlorbenzol; Ester und Lactone wie z.B. Essigsäureethylester, Butyrolacton oder Valerolacton; Carbonsäureamide und Lactame wie z.B. Dimethylformamid, Dimethylacetamid und N-Methylpyrrolidon. Sofern die Reaktanden schwerlöslich sind, können sie in Form von Suspensionen in einem Lösungsmittel eingesetzt werden.

Das Verfahren wird vorteilhaft bei erhöhten Temperaturen durchgeführt, zum Beispiel bei 30 bis 300 °C, bevorzugt bei 50 bis 250 °C. Man kann zum Beispiel so vorgehen, dass man zu einer heissen Lösung von Verbindungen der Formel II eine Lösung der Kupferverbindung zugibt und dann das Reaktionsgemisch abkühlen lässt. Der gebildete kristalline Niederschlag wird danach abfiltriert und gegebenenfalls durch Auswaschen gereinigt und getrocknet. Wenn grössere Kristallite gewünscht sind, kann man eine diffusionskontrollierte Reaktion durchführen, indem man die festen Reaktanden getrennt in den Vorratsbehältern eines Reaktionsgefässes vorlegt und dann mit einem Lösungsmittel überschichtet. Die Radikalkationsalze werden in hoher Reinheit erhalten.

Bevorzugte Verbindungen der Formel I sind (Tetrathiotetracen)Cl_{0,5}, (Tetrathiotetracen)Br_{0,5}, (Tetrathiotetracen)I_{0,75},(Tetraselenotetracen)Cl_{0,5}, (Tetraselenotetracen)Br_{0,5}, (2-Fluor- oder 2,3-Difluortetrathio- oder -tetraselenotetracen)Cl_{0,5}, (2-Fluor- oder 2,3-Difluortetrathio- oder -tetraselenotetracen)Br_{0,5} und (Tetrathiotetracen)(CuCl₂)_{0,4 bis 0,6}. X in Formel I steht bevorzugt für S oder Se und n steht bevorzugt für eine Zahl von 0,3 bis 0,8. Wenn X Cl oder Br bedeutet, steht n bevorzugt für 0,5; wenn X für I steht, bedeutet n bevorzugt eine Zahl von 0,7 bis 0,8, und wenn X für CuCl₂ steht, bedeutet n bevorzugt eine Zahl von 0,4 bis 0,5.

Geeignete inerte Lösungsmittel für Polymere beziehungsweise Ausgangsverbindungen für Polymere sind zum Beispiel polare und bevorzugt aprotische Lösungsmittel, die alleine oder in Mischungen aus mindestens zwei Lösungsmitteln verwendet werden können. Beispiele sind: Ether (Dibutylether, Tetrahydrofuran, Dioxan, Ethylenglykolmonomethyl- oder -dimethylether, Ethylenglykolmonoethyl- oder -diethylether, Diethylenglykoldiethylether, Triethylenglykoldimethylether), halogenierte Kohlenwasserstoffe (Methylenchlorid, Chloroform, 1,2-Dichlorethan, 1,1,1-Trichlorethan, 1,1,2,2-Tetrachlorethan), Carbonsäureester und Lactone (Essigsäureethylester, Propionsäuremethylester, Benzoesäureethylester, 2-Methoxyethylacetat, γ-Butyrolacton, δ-Valerolacton, Pivalolacton), Carbonsäureamide und Lactame (N,N-Dimethylformamid, N,N-Diethylformamid, N,N-Dimethylacetamid, Tetramethylharnstoff, Hexamethylphosphorsäuretriamid, γ-Butyrolactam, ε-Caprolactam, N-Methylpyrrolidon, N-Acetylpyrrolidon, N-Methylcaprolactam), Sulfoxide (Dimethylsulfoxid), Sulfone (Dimethylsulfon, Diethylsulfon, Trimethylensulfon, Tetramethylensulfon), tertiäre Amine (N-Methylpiperidin, N-Methylmorpholin) substituierte Benzole (Benzonitril, Chlorbenzol, o-Dichlorbenzol, 1,2,4-Trichlorbenzol, Nitrobenzol, Toluol, Xylol) und Nitrile (Acetonitril, Propionitril).

Die Lösungmittel können in der Verfahrensstufe a) gleichzeitig als Suspensionsmittel dienen und sie können geringe Mengen, zum Beispiel 0,001 bis 2 Gew.-% eines Dispergiermittels enthalten, bezogen auf die Menge des Lösungsmittels. In dieser Verfahrensstufe können aber auch wässrige oder wässrig organische Systeme als Lösungsmittel bzw. Suspensionsmittel verwendet werden.

Bei den thermoplastischen Kunststoffen kann es sich zum Beispiel um die folgenden Polymeren, Copolymeren bzw. Mischungen von diesen Polymeren handeln:
1. Polymere von Mono- und Diolefinen, beispielsweise Polypropylen, Polyisobutylen, Polybuten- 1, Polymethylpenten- 1, Polyisopren oder Polybutadien sowie Polymerisate von Cycloolefinen wie z.B. von Cyclopenten oder Norbornen; ferner Polyethylen (das gegebenenfalls vernetzt sein kann), z.B. Polyethylen hoher Dichte (HDPE), Polyethylen niederer Dichte (LDPE), lineares Polyethylen niederer Dichte (LLDPE).
2. Mischungen der unter 1) genannten Polymeren, z.B. Mischungen von Polypropylen mit Polyisobutylen, Polypropylen mit Polyethylen (z.B. PP/HDPE, PP/LDPE) und Mischungen verschiedener Polyethylentypen (z.B. LDPE/HDPE).
3. Copolymere von Mono- und Diolefinen untereinander oder mit anderen Vinylmonomeren, wie z.B. Ethylen-Propylen-Copolymere, lineares Polyethylen niederer Dichte (LLDPE) und Mischungen desselben mit Polyethylen niederer Dichte (LDPE), Propylen-Buten-1-Copolymere, Propylen-Isobutylen-Copolymere, Ethylen-Buten- 1-Copolymere, Ethylen-Hexen-Copolymere, Ethylen-Methylpenten-Copolymere, Ethylen-Hepten-Copolymere, Ethylen-Octen-Copolymere, Propylen-Butadien-Copolymere, Isobutylen-Isopren-Copolymere, Ethylen-Alkylacrylat-Copolymere, Ethylen-Alkylmethacrylat- Copolymere, Ethylen-Vinylacetat-Copolymere oder Ethylen-Acrylsäure-Copolymere und deren Salze (Ionomere), sowie Terpolymere von Ethylen mit Propylen und einem Dien, wie Hexadien, Dicyclopentadien oder Ethylidennorbornen; ferner Mischungen solcher Copolymere untereinander und mit unter 1) genannten Polymeren, z.B. Polypropylen/Ethylen-Propylen-Copolymere, LDPE/Ethylen-Vinylacetat-Copolymere, LDPE/Ethylen-Acrylsäure-Copolymere, LLDPE/Ethylen-Vinylacetat-Copolymere und LLDPE/Ethylen-Acrylsäure-Copolymere.
   3a. Kohlenwasserstoffharze (z.B. C₅-C₉) inklusive hydrierte Modifikationen davon (z.B. Klebrigmacherharze).
4. Polystyrol, Poly-(p-methylstyrol), Poly-(α-methylstyrol).
5. Copolymere von Styrol oder α-Methylstyrol mit Dienen oder Acrylderivaten, wie z.B. Styrol-Butadien, Styrol-Acrylnitril, Styrol-Alkylmethacrylat, Styrol-Butadien-Alkylacrylat, Styrol-Maleinsäureanhydrid, Styrol-Acrylnitril-Methylacrylat; Mischungen von hoher Schlagzähigkeit aus Styrol-Copolymeren und einem anderen Polymer, wie z.B. einem Polyacrylat, einem Dien-Polymeren oder einem Ethylen-Propylen-Dien-Terpolymeren; sowie Block-Copolymere des Styrols, wie z.B. Styrol-Butadien-Styrol, Styrol-Isopren-Styrol, Styrol-Ethylen/Butylen-Styrol oder Styrol-Ethylen/Propylen-Styrol.
6. Pfropfcopolymere von Styrol oder α-Methylstyrol, wie z.B. Styrol auf Polybutadien, Styrol auf Polybutadien-Styrol- oder Polybutadien-Acrylnitril-Copolymere, Styrol und Acrylnitril (bzw. Methacrylnitril) auf Polybutadien; Styrol, Acrylnitril und Methylmethacrylat auf Polybutadien; Styrol und Maleinsäureanhydrid auf Polybutadien; Styrol, Acrylnitril und Maleinsäureanhydrid oder Maleinsäureimid auf Polybutadien; Styrol und Maleinsäureimid auf Polybutadien, Styrol und Alkylacrylate bzw. Alkylmethacrylate auf Polybutadien, Styrol und Acrylnitril auf Ethylen-Propylen-Dien-Terpolymeren, Styrol und Acrylnitril auf Polyalkylacrylaten oder Polyalkylmethacrylaten, Styrol und Acrylnitril auf Acrylat-Butadien-Copolymeren, sowie deren Mischungen mit den unter 5) genannten Copolymeren, wie sie z.B. als sogenannte ABS-, MBS-, ASA- oder AES-Polymere bekannt sind.
7. Halogenhaltige Polymere, wie z.B. Polychloropren, Chlorkautschuk, chloriertes oder chlorsulfoniertes Polyethylen, Copolymere von Ethylen und chloriertem Ethylen, Epichlorhydrinhomo- und -copolymere, insbesondere Polymere aus halogenhaltigen Vinylverbindungen, wie z.B. Polyvinylchlorid, Polyvinylidenchlorid, Polyvinylfluorid, Polyvinylidenfluorid; sowie deren Copolymere, wie Vinylchlorid-Vinylidenchlorid, Vinylchlorid-Vinylacetat oder Vinylidenchlorid-Vinylacetat.
8. Polymere, die sich von α,β-ungesättigten Säuren und deren Derivaten ableiten, wie Polyacrylate und Polymethacrylate, Polyacrylamide und Polyacrylnitrile.
9. Copolymere der unter 8) genannten Monomeren untereinander oder mit anderen ungesättigten Monomeren, wie z.B. Acrylnitril-Butadien-Copolymere, Acrylnitril-Alkylacrylat-Copolymere, Acrylnitril-Alkoxyalkylacrylat-Copolymere, Acrylnitril-Vinylhalogenid-Copolymere oder Acrylnitril-Alkylmethacrylat-Butadien-Terpolymere.
10. Polymere, die sich von ungesättigten Alkoholen und Aminen bzw. deren Acylderivaten oder Acetalen ableiten, wie Polyvinylalkohol, Polyvinylacetat, -stearat, -benzoat, -maleat, Polyvinylbutyral, Polyallylphthalat, Polyallylmelamin; sowie deren Copolymere mit in Punkt 1 genannten Olefinen.
11. Homo- und Copolymere von cyclischen Ethern, wie Polyalkylenglykole, Polyethylenoxyd, Polypropylenoxyd oder deren Copolymere mit Bisglycidylethern.
12. Polyacetale, wie Polyoxymethylen, sowie solche Polyoxymethylene, die Comonomere, wie z.B. Ethylenoxid, enthalten; Polyacetale, die mit thermoplastischen Polyurethanen, Acrylaten oder MBS modifiziert sind.
13. Polyphenylenoxide und -sulfide und deren Mischungen mit Styrolpolymeren oder Polyamiden.
14. Polyurethane, die sich von Polyethern, Polyestern und Polybutadienen mit endständigen Hydroxylgruppen einerseits und aliphatischen oder aromatischen Polyisocyanaten andererseits ableiten, sowie deren Vorprodukte.
15. Polyamide und Copolyamide, die sich von Diaminen und Dicarbonsäuren und/oder von Aminocarbonsäuren oder den entsprechenden Lactamen ableiten, wie Polyamid 4, Polyamid 6, Polyamid 6/6, 6/10, 6/9, 6/12, 4/6, Polyamid 11, Polyamid 12, aromatische Polyamide ausgehend von m-Xylylendiamin und Adipinsäure; Polyamide, hergestellt aus Hexamethylendiamin und Iso- und/oder Terephthalsäure und gegebenenfalls einem Elastomer als Modifikator, z.B. Poly-2,4,4-trimethylhexamethylenterephthalamid, Poly-m-phenylen-isophthalamid. Block-Copolymere der vorstehend genannten Polyamide mit Polyolefinen, Olefin-Copolymeren, Ionomeren oder chemisch gebundenen oder gepfropften Elastomeren; oder mit Polyethern, wie z.B. mit Polyethylenglykol, Polypropylenglykol oder Polytetramethylenglykol. Ferner mit EPDM oder ABS modifizierte Polyamide oder Copolyamide; sowie während der Verarbeitung kondensierte Polyamide ("RIM-Polyamidsysteme").
16. Polyharnstoffe, Polyimide, Polyamid-imide und Polybenzimidazole.
17. Polyester, die sich von Dicarbonsäuren und Dialkoholen und/oder von Hydroxycarbonsäuren oder den entsprechenden Lactonen ableiten, wie Polyethylenterephthalat, Polybutylenterephthalat, Poly-1,4-dimethylolcyclohexanterephthalat, Polyhydroxybenzoate, sowie Block-Polyether-ester, die sich von Polyethern mit Hydroxylendgruppen ableiten; ferner mit Polycarbonaten oder MBS modifizierte Polyester.
18. Polycarbonate und Polyestercarbonate.
19. Polysulfone, Polyethersulfone und Polyetherketone.
20. Polyether aus Diglycidylverbindungen, zum Beispiel Diglycidylethern und Diolen, z. B. aus Bisphenol-A-Diglycidylether und Bisphenol-A.
21. Natürliche Polymere, wie Cellulose, Naturkautschuk, Gelatine, sowie deren polymerhomolog chemisch abgewandelte Derivate, wie Celluloseacetate, -propionate und -butyrate, bzw. die Celluloseether, wie Methylcellulose; sowie Kolophoniumharze und Derivate.
22. Mischungen (Polyblends) der vorgenannten Polymeren, wie z.B. PP/EPDM, Polyamid/EPDM oder ABS, PVC/EVA, PVC/ABS, PVC/MBS, PC/ABS, PBTP/ABS, PC/ASA, PC/PBT, PVC/CPE, PVC/Acrylate, POM/thermoplastisches PUR, PC/thermoplastisches PUR, POM/Acrylat, POM/MBS, PPO/HIPS, PPO/PA 6.6 und Copolymere, PA/HDPE, PA/PP, PA/PPO.

Bevorzugte Thermoplaste sind Polyolefine, Polystyrol, Polyvinylchlorid, Polyvinylidenchlorid, Polyvinylidenfluorid, Polyacrylate, Polymethacrylate, Polyamide, Polyester, Polycarbonate, aromatische Polysulfone, aromatische Polyether, aromatische Polyethersulfone, Polyimide und Polyvinylcarbazol.

Bei den Ausgangsverbindungen für duroplastische Kunststoffe kann es sich um solche Verbindungen handeln, die zum Beispiel zu folgenden Duroplasten und strukturell vernetzten Polymeren führen:
1. Vernetzte Polymere, die sich von Aldehyden einerseits und Phenolen, Harnstoff oder Melamin andererseits ableiten, wie Phenol-Formaldehyd-, Harnstoff-Formaldehyd- und Melamin-Formaldehydharze.
2. Trocknende und nicht-trocknende Alkydharze.
3. Ungesättigte Polyesterharze, die sich von Copolyestern gesättigter und ungesättigter Dicarbonsäuren mit mehrwertigen Alkoholen, sowie Vinylverbindungen als Vernetzungsmittel ableiten, wie auch deren halogenhaltige, schwerbrennbare Modifikationen.
4. Vernetzbare Acrylharze, die sich von substituierten Acrylsäureestern ableiten, wie z.B. von Epoxyacrylaten, Urethan-acrylaten oder Polyester-acrylaten.
5. Alkydharze, Polyesterharze und Acrylatharze, die mit Melaminharzen, Harnstoffharzen, Polyisocyanaten oder Epoxidharzen vernetzt sind.
6. Kautschuk auf der Basis von vernetzten Polydienen, zum Beispiel Butadien oder Isopren; Silikonkautschuk.
7. Epoxidharze, die sich von Polyepoxiden ableiten, z.B. von Bisglycidylethern von Polyolen oder von cycloaliphatischen Diepoxiden, und die gegebenenfalls einen Härter als Vernetzungsmittel enthalten, oder die unter Verwendung von Härtungsbeschleunigern thermisch oder durch Einwirkung von Strahlung vernetzt sind.

Unter den vernetzten Polymeren sind vernetzte Epoxidharze bevorzugt, denen als Polyepoxide bevorzugt Glycidylverbindungen mit durchschnittlich zwei Epoxidgruppen im Molekül zu Grunde liegen. Als Glycidylverbindungen kommen vor allem solche mit zwei an ein Heteroatom (z.B. Schwefel, vorzugsweise Sauerstoff oder Stickstoff) gebundenen Glycidylgruppen, β-Methylglycidylgruppen oder 2,3-Epoxycyclopentylgruppen in Frage; genannt seien insbesondere Bis-(2,3-epoxycyclopentyl)-ether; Diglycidylether von mehrwertigen aliphatischen Alkoholen, wie 1,4-Butandiol, oder Polyalkylenglykolen, wie Polypropylenglykole; Diglycidylether von cycloaliphatischen Polyolen, wie 2,2-Bis-(4-hydroxycyclohexyl)-propan; Diglycidylether von mehrwertigen Phenolen, wie Resorcin, Bis-(p-hydroxyphenyl)-methan, 2,2-Bis-(p-hydroxyphenyl)-propan (=Diomethan), 2,2-Bis-(4'-hydroxy-3',5'-dibromphenyl)-propan, 1,3-Di-(p-hydroxyphenyl)-ethan; Di-(β-methylglycidyl)-ether der oben angeführten zweiwertigen Alkohole oder zweiwertigen Phenole; Diglycidylester von Dicarbonsäuren, wie Phthalsäure, Terephthalsäure, Δ₄-Tetrahydrophthalsäure und Hexahydrophthalsäure; N,N-Diglycidylderivate von primären Aminen und Amiden und heterocyclischen, zwei N-Atome enthaltenden Stickstoffbasen, und N,N'-Diglycidylderivate von disekundären Diamiden und Diaminen, wie N,N-Diglycidylanilin, N,N-Diglycidyltoluidin, N,N-Diglycidyl-p-aminophenyl-methyl-ether, N,N'-Dimethyl-N,N'-diglycidyl-bis-(p-aminophenyl)-methan; N',N''-Diglycidyl-N-phenyl-isocyanurat; N,N'-Diglycidylethylenharnstoff; N,N'-Diglycidyl-5,5-dimethyl-hydantoin, N,N'-Diglycidyl-5-isopropyl-hydantoin, N,N-Methylen-bis-(N',N'-diglycidyl-5,5-dimethylhydantoin), 1,3-Bis-(N-glycidyl-5,5-dimethylhydantoin)-2-hydroxypropan; N,N'-Diglycidyl5,5-dimethyl-6-isopropyl-5,6-dihydro-uracil, Triglycidylisocyanurat.

Eine bevorzugte Gruppe von Epoxidharzen sind glycidylierte Novolake, Hydantoine, Aminophenole, Bisphenole und aromatische Diamine oder cycloaliphatische Epoxidverbindungen. Besonders bevorzugte Epoxidharze sind glycidylierte Kresolnovolake, Bisphenol-A- und Bisphenol-F-diglycidylether, Hydantoin-N,N'-bisglycid, p-Aminophenoltriglycid, Diaminodiphenylmethantetraglycid, Vinylcyclohexendioxid, 3,4-Epoxycyclohexylmethyl-3,4-epoxycyclohexancarboxylat oder Mischungen hiervon.

Geeignet sind auch vorreagierte Addukte solcher Epoxidverbindungen mit Epoxidhärtern, zum Beispiel ein Addukt aus Bisphenol-A-diglycidylether und Bisphenol-A, oder mit Oligoestern mit zwei terminalen Carboxylgruppen und Epoxiden vorreagierte Addukte.

Als Härter für Epoxidharze kommen saure oder basische Verbindungen in Frage. Als geeignete Härter seien zum Beispiel genannt: mehrwertige Phenole (Resorcin, 2,2-Bis-(4-hydroxyphenyl)-propan) oder Phenol-Formaldehyd-Harze; mehrbasische Carbonsäuren und ihre Anhydride, z. B. Phthalsäureanhydrid, Tetrahydrophthalsäureanhydrid, Hexahydrophthalsäureanhydrid, 4-Methylhexahydrophthalsäureanhydrid, 3,6-Endomethylen-tetrahydrophthalsäureanhydrid, 4-Methyl-3,6-endomethylen-tetrahydrophthalsäureanhydrid (Methylnadicanhydrid), 3,4,5,6,7,7-Hexachlor-endomethylen-tetrahydrophthalsäureanhydrid, Bernsteinsäureanhydrid, Adipinsäureanhydrid, Trimethyladipinsäureanhydrid, Sebacinsäureanhydrid, Maleinsäureanhydrid, Dodecylbernsteinsäureanhydrid, Pyromellitsäuredianhydrid, Trimellitsäureanhydrid, Benzophenontetracarbonsäuredianhydrid, oder Gemische solcher Anhydride.

Eine bevorzugte Gruppe von Härtern sind Novolake und Polycarbonsäureanhydride.

Die Epoxidharze können auch zusätzlich mit Härtungsbeschleunigern oder nur mit thermischen Härtungskatalysatoren gehärtet werden. Beispiele sind 3-Ethyl-4-methylimidazol, Triamylammoniumphenolat); Mono- oder Polyphenole (Phenol, Diomenthan, Salicylsäure); Bortrifluorid und seine Komplexe mit organischen Verbindungen wie z. B. Bortrifluorid-Etherkomplexe und Bortrifluorid-Amin-Komplexe (BF₃-Monoethylamin-Komplex); Phosphorsäure und Triphenylphosphit.

Härtungsbeschleuniger und Katalysatoren werden üblicherweise in einer Menge von 0,1 bis 10 Gew.-% zugegeben, bezogen auf das Epoxidharz. Härter für Epoxidharze werden im allgemeinen in äquimolaren Mengen verwendet, bezogen auf die Epoxidgruppen und funktionellen Gruppen eines Härters.

Die Suspensionen der Verfahrensstufe a) können auch strahlungsempfindliche Materialien gelöst enthalten, aus denen durch Bestrahlung unter einer Photomaske und anschliessende Entwicklung Reliefbilder erhältlich sind. Es kann sich um Positiv- oder Negativsysteme handeln. Solche Materialien sind z. B. von G.E. Green in J. Macro. Sci.-Revs. Macr. Chem., C21(2), 187-273 (1981-82) und von G. A. Delzenne in Adv. Photochem., 11, S. 1-103 (1979) beschrieben. Die strahlungsempfindlichen Materialien können alleine oder zusammen mit einem Bindemittel, zum Beispiel mindestens einem der zuvor genannten Polymeren, eingesetzt werden. Es handelt sich hauptsächlich um photopolymerisierbare oder photovernetzbare Systeme, die im allgemeinen Photosensibilisatoren und/oder Photoinitiatoren enthalten. Es kann sich zum Beispiel um nichtflüchtige monomere, oligomere oder polymere Substanzen mit photopolymerisierbaren oder photodimerisierbaren ethylenisch ungesättigten Gruppen, um kationisch härtbare Systeme oder um photovernetzbare Polyimide handeln. Solche Polyimide sind zum Beispiel in den EP-A-0 132 221, EP-A-0 134 752, EP-A0 162 017, EP-A-0 181 837 und EP-A-0 182 745 beschrieben.

Den erfindungsgemässen zu verwendenden Suspensionen können weitere Additive zur Verbesserung der Verarbeitungseigenschaften, der mechanischen, elektrischen und thermischen Eigenschaften, der Oberflächeneigenschaften und der Lichtstabilität von Kunststoffen einverleibt sein, zum Beispiel feinteilige Füllstoffe, Verstärkerfüllstoffe, Weichmacher, Gleit- und Entformungsmittel, Haftvermittler, Antistatika, Antioxidantien, Wärme- und Lichtstabilisatoren, Pigmente und Farbstoffe.

In der Verfahrensstufe b) können die gleichen Lösungsmittel, Kunststoffe und Zusätze verwendet werden wie in der Verfahrensstufe a). Die Verbindungen der Formel II sind bekannt und zum Beispiel in den eingangs erwähnten veröffentlichten europäischen Patentanmeldungen beschrieben. Für R₁ bis R₄ und X in den Verbindungen der Formel II gelten die gleichen Bevorzugungen wie für die Verbindungen der Formel I.

Wärmezufuhr bedeutet zum Beispiel eine Temperatur von Raumtemperatur bis zu 350°C, insbesondere 50 bis 200°C. Die Wärme kann vor, während und/oder nach dem Sprühen zugeführt werden. Es hat sich als zweckmässig erwiesen, die Temperatur der Suspensionen im Verfahren a) auf 20 bis 350 °C, bevorzugt 20 bis 200 °C, und die Temperatur der Lösung im Verfahren b) auf 50 bis 350 °C, bevorzugt 50 bis 200 °C einzustellen. Das Mengenverhältnis von Cl-, Br- oder I-haltiger Verbindung zur Verbindung der Formel II beträgt bevorzugt 10:1 bis 1:5, besonders 5:1 bis 1:3 und insbesondere 2:1 bis 1:2. Die halogenhaltige organische Verbindung kann aber auch in erheblich höheren Mengen enthalten sein und gleichzeitig als Lösungsmittel für das thermoplastische Polymer oder eine Ausgangsverbindung für einen duroplastischen Kunststoff und die Verbindung der Formel II dienen, wenn es sich z.B. um eine flüssige halogenhaltige organische Verbindung handelt. Die organische Verbindung kann auch fest sein und sie soll mit dem Polymer mischbar und verträglich sein.

Bei der Cl-, Br- oder I-haltigen organischen Verbindung kann es sich um eine halogenierte, gesättigte oder ungesättigte, aliphatische, cycloaliphatische, aliphatisch-heterocyclische, aromatische oder heteroaromatische organische Verbindung handeln, die durch -CN, HO-, =O, C₁-C₄-Alkyl, C₁-C₄-Alkoxy, -CO-C₁-C₄-Alkyl, -COOC₁-C₄-Alkyl substituiert sein können. Die Halogenverbindungen können einzeln oder in Gemischen eingesetzt werden. Die organische Verbindung ist bevorzugt chloriert und/oder bromiert. Die Verbindungen können einfach halogeniert sein, wie z.B. N-bromierte oder N-chlorierte Dicarbonsäureimide. C-halogenierte Verbindungen weisen zweckmässig einen höheren Halogenierungsgrad auf; bevorzugt sind diese Verbindungen zu mindestens 80 % C-halogeniert, besonders C-bromiert und/oder C-chloriert. Verbindungen, deren Halogenatome durch elektronenziehende Gruppen aktiviert sind, sind besonders günstig. Beispiele für halogenierte organische Verbindungen sind Tetrabrommethan, Bromoform, Trichlorbrommethan, Hexachlorpropen, Hexachlorcyclopropan, Hexachlorcyclopentadien, Hexachlorethan, N-Chlorsuccinimid, Octachlorpropan, n-Octachlorbutan, n-Decachlorbutan, Tetrabromethan, Hexabromethan, Tetrabrom-o-benzochinon, 2,4,4,6-Tetrabrom-2,5-cyclohexadienon, Hexabrombenzol, Chloranil, Hexachloraceton, 1,4,5,6,7,7-Hexachlor-5-norbornen-2,3-dicarbonsäure, 1,2,5,6,9,10-Hexabromcyclododecan, Tetrachlorethylen, Perchlorcyclopentadien, Perchlorbutadien, Dichloracetaldehyd-diethylacetal, 1,4-Dichlor-2-buten, 1,3-Dichlor-2-buten, 3,4-Dichlor-1-buten, Tetrachlorcyclopropen, 1,3-Dichloraceton, 2,3,5,6-Hexachlor-p-xylol, 1,4-Bis(trichlormethyl)-benzol, 1,3-Dibrompropan, 1,6-Dibromhexan, 3-Chlorpropionsäureethylester, 3-Chlortoluol, 2-Chlorpropionsäuremethylester, 2-Chloracrylnitril, Trichloressigsäureethylester, 1,2,3-Trichlorpropan, 1,1,2-Trichlorethan, Chlorameisensäurebutylester, Trichlorethylen, 2,3-Dichlormaleinsäureanhydrid, 1,12-Dibromdodecan, α,α'-Dibrom-p-xylol, α,α'-Dichlor-o-xylol, Phenacylchlorid oder -bromid, 1,10-Dibromdecan, α,α'-Dichlor-p-xylol, α,α'-Dibrom-m-xylol, Jodacetonitril, 2,3-Dichlor-5,6-dicyanobenzochinon, 2,3-Dichlorpropionsäuremethylester, 1-Brom2-chlorethan, 1-Brom-2-chlorpropan, Chlorameisensäure-2-bromethylester, Iodessigsäureethylester, N-Chlor-, N-Brom- oder N-Iodsuccinimid oder -phthalsäureimid, oder Mischungen hiervon. Die organische Verbindung kann auch Tri(C₁-C₆-Alkyl)ammoniumhydrochlorid sein, wobei zusätzlich wässrige H₂O₂-Lösung mitverwendet wird.

Oligomere und polymere Cl-, Br- und I-haltige Verbindungen sind zum Beispiel in der EP-A-0 362 142 beschrieben. Es kann sich um Verbindungen handeln, die in einem Rückgrat aus mindestens zwei Monomereinheiten, zum Beispiel 2 bis 10000, bevorzugt 2 bis 5000 Einheiten, über eine Gruppe -O-, -O-CO-, -CO-R₅-OCO- oder -CO-O- gebundene aliphatische oder cycloaliphatische Seitengruppen enthält, die in α-, β-, γ- oder ω-Stellung mit mindestens einem Cl-, Br- oder I-Atom substituiert sind, und wobei R₅ unsubstituiertes oder mit OH substituiertes C₁-C₁₂-Alkylen, C₄-C₁₂-Cycloalkylen, Benzylen oder Xylylen darstellt. Die aliphatischen und cycloaliphatischen Seitengruppen sind bevorzugt in α-, β- oder γ-Stellung, besonders bevorzugt in α- oder β-Stellung mit Cl, Br oder I substituiert. Die Seitengruppe enthält bevorzugt 1 bis 4 C-Atome, die teilweise oder vollständig mit Cl, Br oder I substituiert ist, wobei die Substitution mit Cl bevorzugt ist. Besonders bevorzugt sind mit Cl substituiertes Methyl oder Ethyl, zum Beispiel -CH₂Cl, -CHCl₂, -CCl₃, -CHClCH₃, -CCl₂CH₃, -CHClCH₂Cl, -CCl₂CH₂Cl, -CHClCHCl₂, -CCl₂CHCl₂, -CCl₂CCl₃, -CH₂CH₂Cl, -CH₂CHCl₂, und -CH₂CCl₃.

Bevorzugte Oligomere und Polymere sind solche mit Strukturelementen der Formel III,

-ORO-CH₂CH(OR₆)CH₂- (III),

worin R₆ einen Cl, Br oder I enthaltenden Rest der Formel C₁-C₄-Alkyl-CO bedeutet, und R einen divalenten organischen Rest mit bevorzugt 2 bis 20 C-Atomen darstellt, zum Beispiel C₂-C₂₀-Alkylen, C₅-C₁₂-Cycloalkylen oder C₆-C₂₀-Arylen, die unsubstituiert oder mit C₁-C₆-Alkyl oder -Alkoxy oder Cl oder Br substituiert sind. Bevorzugte Reste R leiten sich von Alkyliden- oder Cycloalkylidenbisphenolen ab, zum Beispiel von Bisphenol-A oder Bisphenol-F.

Bei den Oligomeren und Polymeren kann es sich auch um strahlungsempfindliche Cl-, Br- oder I-haltige Substanzen handeln, wie sie zum Beispiel in der EP-A-0 362 143 beschrieben sind.

Die oligomeren und polymeren Substanzen können alleine oder zusammen mit mindestens einem der zuvor beschriebenen Polymeren als Bindemittel eingesetzt werden.

Die Cl-, Br- und I-haltigen Verbindungen sowie die Kupferdihalogenide reagieren mit den Verbindungen der Formel II unter Wärmezufuhr zu nadelförmigen Radikalkationensalzen der Formel I, wobei die Kristallnadeln gleichzeitig Netzwerke ausbilden.

Das erfindungsgemässe Verfahren a) kann zum Beispiel so durchgeführt werden, dass man eine Suspension der Radikalkationensalze der Formel I herstellt, gegebenenfalls mit einem im Suspensionsmittel gelösten thermoplastischen Kunststoff oder mindestens einem Ausgangsstoff für duroplastische Kunststoffe, danach die Suspension auf das gegebenenfalls vorerwärmte Trägermaterial sprüht und danach das Suspensionsmittel (Lösungsmittel) unter Wärmeeinwirkung verdampft, zum Beispiel bei 50 bis 250 °C. Wenn mehrere Ausgangsstoffe für Duroplaste verwendet werden, können diese in einer oder in getrennten Lösungen eingesetzt werden, wobei im letzteren Fall die Vermischung im Düsenkopf erfolgen kann. Die Ausgangsstoffe für Duroplaste werden nach dem Abdampfen durch Wärmeeinwirkung ausgehärtet.

Das Suspensionsverfahren bietet den Vorteil, dass Korrosionsprobleme bei den verwendeten Einrichtungen weitgehend vermieden werden können, da keine Redoxsysteme eingesetzt werden, wie beim Verfahren b).

Bei dem Verfahren b) können die einzelnen Komponenten gemeinsam gelöst oder getrennte Lösungen hergestellt werden, die man vor der Durchführung des Verfahrens vereinigt. Die Vereinigung kann in einem Vorratsgefäss oder im Düsenkopf vorgenommen werden. Mit der Vereinigung der reaktiven Komponenten kann bereits die Bildung von Radikalkationensalzen der Formel I beginnen, wobei Suspensionen entstehen. Die Bildung kann aber auch erst in den Sprühtropfen erfolgen. Ein wesentlicher Vorteil des erfindungsgemässen Verfahrens besteht in der Möglichkeit, die Grösse der Kristallnadeln und die Maschenweite der Nadelnetzwerke durch verschiedene Parameter gezielt zu beeinflussen, zum Beispiel Temperaturen (der Lösungen, beim Abdampfen und beim vorerwärmten Trägermaterial) Konzentrationen der Polymeren und Radikalkationensalze beziehungsweise der Verbindungen der Formel II, Viskositäten der Sprühlösungen, Druck und Grösse der Düsenöffnungen beziehungsweise der Tröpfchengrösse.

Die Verdampfung des Lösungsmittels wird im allgemeinen bei Temperaturen vorgenommen, die für die gleichzeitige Bildung der Radikalkationensalze ausreichen, zum Beispiel 50 bis 250 °C.

Bei Verwendung ebener Trägermaterialien können die Beschichtungen nach dem Abkühlen abgelöst und so freitragende Filme oder Folien hergestellt werden. Reine Nadelnetzwerke können mit einer Schutzschicht aus Kunsstoffen versehen und danach gegebenenfalls vom Trägermaterial abgelöst werden.

Die Schichtdicken können in einem breiten Rahmen variieren, je nach Länge und Wiederholungen des Beschichtungsverfahrens. Sie können zum Beispiel 0,01 bis 5000 »m, bevorzugt 0,1 bis 1000 »m und besonders bevorzugt 0,1 bis 500 »m betragen. In einem kurzen Arbeitsgang können zum Beispiel Schichtdicken von 0,01 bis 20 »m erzielt werden.

Mit dem erfindungsgemässen Verfahren werden je nach Wahl des Polymers opake oder transparente Beschichtungen erhalten, die hervorragende elektrische Eigenschaften aufweisen. So weisen die Beschichtungen und Filme oder Folien überraschend eine ausgezeichnete Entladungsfähigkeit auf, die für heterogene Materialien sonst schwierig oder gar nicht zu erreichen ist. Die Beschichtungen eignen sich daher besonders zur antistatischen Ausrüstung von Formteilen oder für die elektrostatische Abschirmung von Bauteilen und Formkörpern. Die hohen elektrischen Leitfähigkeiten ermöglichen ferner die Verwendung als elektrische Leiter, zum Beispiel als Elektroden für Displayelemente oder elektronische Bauteile. Die Zusammensetzungen weisen auch hervorragende mechanische Festigkeiten und mechanische Gebrauchseigenschaften auf. Die Beschichtungen eignen sich ferner als leitende Schichten zur Potentialglättung von Hochspannungskabeln, als elektrostatische Beschichtungen von Bildschirmen und als farbige elektrostatische Farbbeschichtungen. Ein besonderer Vorteil des erfindungsgemässen Verfahrens besteht in den Möglichkeiten, grossflächige Beschichtungen vorzunehmen und räumlich gestaltete Formkörper vollständig zu beschichten.

Die nachfolgenden Beispiele erläutern die Erfindung näher.

### A) Herstellungsbeispiel

Beispiel A1: 60 mg Tetrathiotetracen (TTT) werden in 33 g γ-Butyrolacton unter Argonatmosphäre bei 180 °C Badtemperatur mit Rühren gelöst (grüne Lösung). Anschliessend wird eine gelbe Lösung von 15,3 mg CuCl₂·2 H₂O in 5 g γ-Butyrolacton zugegeben. Die Lösung wird sofort dunkelviolett. Der Rührer und die Heizung des Oelbads werden abgestellt und die Reaktionslösung wird dann im Ölbad abgekühlt. Dabei kristallisieren Nadeln aus (Ausbeute 57 mg). Der spezifische Widerstand der Kristalle beträgt 0.5 bis 1 Ω·cm. Die Röntgenstrukturanalyse zeigt, dass es sich um das Radikalkationensalz der Zusammensetzung TTT(CuCl₂)_{0,45} handelt.

### B) Anwendungsbeispiele

Beispiel B1: 9,0 mg TTT und 0,6 g Polycarbonat werden bei 160 °C in 18 g γ-Butyrolacton gelöst und mit einer Lösung von 2,1 mg CuCl₂·2 H₂O in 2 g γ-Butyrolacton vermischt. Das Reaktionsgemisch wird auf eine vorgeheizte Glasplatte gesprüht (Sprühbedingungen: Glasdüse mit einem Durchmesser von 1 mm, Treibgas Argon, Abstand von Sprühdüse zur Glasplatte ca. 15 cm). Nach Abdampfen des Lösungsmittels bei 130 °C bleibt eine 8 »m dicke Schicht mit einem feinen Nadelnetzwerk aus elektrisch leitenden Kristallnadeln von TTT(CuCl₂)_{0,48} in einer Polycarbonatmatrix zurück. Der spezifische Widerstand beträgt 2,4·10³ Ωcm. Die Schichtdicke beträgt etwa 5 »m.

Beispiel B2: 9,0 mg Tetrathiotetracen und 0,6 g Polycarbonat werden bei 150 °C in 16 g Anisol gelöst. Dazu werden 2,0 mg CuCl₂·2 H₂O gelöst in 2 g γ-Butyrolacton gegeben. Das Radikalkationensalz kristallisiert sofort in kleinen Nadeln aus. Das Reaktionsgemisch wird auf eine vorgeheizte Glasplatte gesprüht (Sprühbedingungen wie bei Beispiel B1) und das Lösungsmittel bei 100 °C verdampft. Der gebildete Polycarbonatfilm ist transparent und enthält ein feines, dichtes, elektrisch leitendes Nadelnetzwerk. Der spezifische Widerstand beträgt 6·10³ Ωcm. Die Schichtdicke beträgt etwa 4 »m.

Beispiel B3: 17,9 mg Tetraselenotetracen und 0,9 g Polycarbonat werden in 24 g N-Methylpyrrolidon bei 130 °C gelöst. Nach 25 Minuten werden 40 »l Hexachlorpropen zugegeben. Das Reaktionsgemisch wird kurz verrührt und dann auf eine vorgeheizte Glasplatte gesprüht (Zweistoffdüse aus Stahl, Argon als Treibmittel, Abstand Düse zur Glasplatte etwa 20 cm). Danach wird das Lösungsmittel bei 100 °C verdampft. Der Polycarbonatfilm ist transparent und enthält ein feines und dichtes, elektrisch leitendes Nadelnetzwerk. Der spezifische Widerstand beträgt 1,4 Ω·cm. Die Schichtdicke beträgt etwa 5 »m.

Beispiel B4:
8,0 mg Tetraselenotetracen und 0,6 g Polycarbonat werden bei 160 °C in 20 g γ-Butyrolacton gelöst. Danach werden 8 »l Hexachlorpropen zugegeben und das Gemisch auf eine vorgeheizte Glasplatte gesprüht (Glasdüse mit einem Durchmesser von 1 mm, Treibgas Argon, Abstand Düse/Glasplatte 15 cm). Nach dem Abdampfen des Lösungsmittels bei 100 °C verbleibt ein transparenter Film mit einem feinen Kristallnadelnetzwerk aus (Tetraselenotetracen)₂Cl in der Polycarbonatmatrix. Der spezifische Widerstand beträgt 90 Ω·cm. Die Schichtdicke beträgt 5 »m.

Beispiel B5: 15,27 mg Tetrathiotetracen und 3,75 g eines Polyethers aus Bisphenol-A-Diglycidylether und Bisphenol-A werden bei 150 °C in 60 g Anisol gelöst. Nach etwa 30 Minuten werden 2,5 g einer Lösung von 3,75 mg CuCl₂·2 H₂O und 2% Wasser in 2,5 g γ-Butyrolacton und 750 »l einer Anisollösung enthaltend 10% eines Polyurethanoligomers (Netzmittel) in Xylol zugegeben und vermischt. Das Reaktionsgemisch wird auf eine vorgeheizte Glasplatte gesprüht (Sprühbedingungen: Zweistoffdüse aus Stahl, Treibgas Argon, Abstand von Sprühdüse zur Glasplatte ca. 20 cm, Spraygeschwindigkeit 4 cm/s). Nach Abdampfen des Lösungsmittels bei 50 °C bleibt eine 5 »m dicke Schicht mit einem dichten Nadelnetzwerk aus elektrisch leitenden Kristallnadeln von TTT(CuCl₂)_{0,43} in einer Polyethermatrix zurück. Der spezifische Widerstand beträgt 2·10³ Ωcm.

Beispiel B6: 44,18 mg Tetraselenotetracen und 3 g Polyether aus Bisphenol-A-Diglycidylether und Bisphenol-A werden bei 150 °C in 48 g N-Methylpyrrolidon (NMP)gelöst. Nach etwa 45 Minuten 400 »l NMP-Lösung enthaltend 1% Trimethylammoniumhydrochlorid und 2% Wasser, und anschliessend 300 »l einer H₂O₂-Lösung in NMP/Wasser (9:1) zugegeben und vermischt. Man sprayt das Gemisch auf eine Glasplatte (Sprühbedingungen: Zweistoffdüse aus Stahl, Treibgas Argon, Abstand von Sprühdüse zur Glasplatte ca. 20 cm, Spraygeschwindigkeit 4 cm/s). Nach Abdampfen des Lösungsmittels bei 100 °C bleibt eine 5 »m dicke Schicht mit einem dichten Nadelnetzwerk aus elektrisch leitenden Kristallnadeln von (Tetraseleno)₂Cl in einer Polyethermatrix zurück. Die Leitfähigkeit beträgt 1 S/cm. Die Haftung der Schicht auf der Glasunterlage ist ausgezeichnet.

Beispiel B7: Messung der Oberflächenentladung.
Die Oberflächenspannung von etwa 200 V wird mit Hilfe eines goldbeschichteten Wolframdrahts von 50 Micrometer Durchmesser gebildet, der mit einer Spannung von ungefähr 3.4 kV aufgeladen wird. Diese Spannung ist so geregelt, dass der Strom bei konstant 20 nA pro cm Drahtlänge gehalten wird. Die Probe wird mit Silberpaste auf einen Glasträger geklebt und mit einer Kontaktspitze am Rand des Trägers elektrisch verbunden. Während eines Messverlaufs verschiebt sich der Träger 8 mm unter dem Coronadraht mit einer Geschwindigkeit von etwa 50 cm/s, und hält an dem Punkt an, wo die Kontaktspitze in einen geerdeten leitfähigen Schaum eintaucht. Die Probe liegt dann unter einem Feldmeter (Isoprobe Electrostatic Voltmeter 244, Monroe Electronics Inc.). Der Abfall der gemessenen Oberflächenspannung wird mit einem digitalen Oszilloskop gespeichert. Die Folien der Beispiele B1, B2 und B4 werden mit diesem Test geprüft. Die Oberflächenspannung wird 0,5 Sekunden nach der Coronaaufladung gemessen. Ergebnis:
Beispiel B1: 2 ± 1 Volt; Beispiel B2: 2 ± 1 Volt; Beispiel B4: 3 ± 1 Volt.

## Patentansprüche

1. Verfahren zur Herstellung einer elektrisch leitenden Schicht auf einem Tragermaterial, bei dem die elektrisch leitende Schicht aus einem Netzwerk von Kristallnadeln elektrisch leitender Radikalkationensalze der Formel (I) 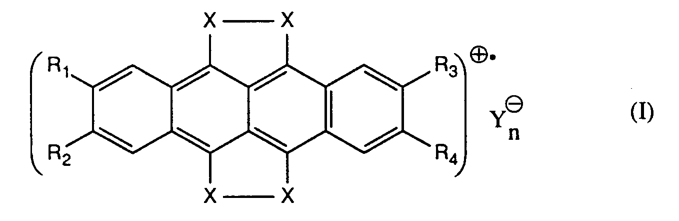 besteht und das Netzwerk in einer Polymermatrix eingebettet sein kann, worin X für S, Se oder Te steht, n eine Zahl von 0,3 bis 0,9 bedeutet, R₁ für H, F, Cl oder CH₃ und R₂, R₃ und R₄ für H stehen, oder R₁, R₂, R₃ und R₄ jeweils F oder CH₃ darstellen, oder R₁ und R₂ jeweils F, Cl oder CH₃ und R₃ und R₄ jeweils H bedeuten, oder R₁ und R₃ oder R₁ und R₄ jeweils F und R₂ und R₄ oder R₂ und R₃ jeweils H darstellen, und Y Cl, Br, I oder CuCl₂ bedeutet, durch Beschichten mindestens einer Oberfläche des Trägermaterials mit
a) einer Suspension von Kristallnadeln der Radikalkationensalze der Formel I in einem inerten Lösungsmittel, das zusätzlich einen thermoplastischen Kunststoff oder mindestens eine Ausgangsverbindung für einen duroplastischen Kunststoff gelöst enthalten kann, oder
b) einer Lösung von (b1) einer Verbindung der Formel II 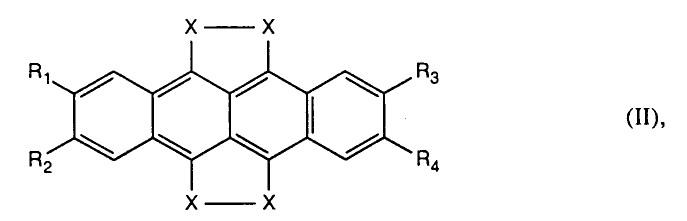 worin R₁, R₂, R₃, R₄ und X die zuvor angegebenen Bedeutungen haben, (b2) einer monomeren, oligomeren oder polymeren und Cl-, Br- oder I-haltigen organischen Verbindung, die unter Wärmeeinwirkung mit einer Verbindung der Formel II eine Verbindung der Formel I bildet, oder wasserfreiem CuCl₂, einem CuCl₂-Aquokomplex oder einem CuCl₂-Lösungsmittelkomplex, und (b3) gegebenenfalls einem thermoplastischen Kunststoff oder mindestens einer Ausgangsverbindung für einen duroplastischen Kunststoff, in einem inerten Lösungsmittel und anschliessendem Verdampfen des Lösungsmittels,
dadurch gekennzeichnet, dass die Schicht mittels Sprühen durch Düsen auf das Trägermaterial aufgebracht wird.

2. Verfahren gemäss Anspruch 1, dadurch gekennzeichnet, dass der Durchmesser der Düsenöffnungen von 0,1 bis 10 mm beträgt.

3. Verfahren gemäss Anspruch 1, dadurch gekennzeichnet, dass der Druck für das Sprühen durch Treibgase erzeugt wird.

4. Verfahren gemäss Anspruch 3, dadurch gekennzeichnet, dass als Treibgase Argon oder Stickstoff verwendet werden.

5. Verfahren gemäss Anspruch 3, dadurch gekennzeichnet, dass der Druck der Treibgase 10⁴ bis 10⁶ Pa beträgt.

6. Verfahren gemäss Anspruch 1, dadurch gekennzeichnet, dass die Temperatur des Düsenkopfes von Raumtemperatur bis 250 °C beträgt.

7. Verfahren gemäss Anspruch 1, dadurch gekennzeichnet, dass der Abstand der Düsenköpfe zum Trägermaterial von 1 bis 100 cm beträgt.

8. Verfahren gemäss Anspruch 1, dadurch gekennzeichnet, dass ein Düsenkopf oder mehrere Düsenköpfe gleichzeitig verwendet werden.

9. Verfahren gemäss Anspruch 1, dadurch gekennzeichnet, dass die Konzentration der Verbindungen der Formel I und II 0,1 Gew.-% bis 20 Gew.-% beträgt, bezogen auf die Suspension beziehungsweise Lösung.

10. Verfahren gemäss Anspruch 1, dadurch gekennzeichnet, dass die Konzentration der thermoplastischen Kunststoffe oder Ausgangsverbindungen für duroplastische Kunststoffe in der Lösung bis zu 60 Gew.-% beträgt.

11. Verfahren gemäss Anspruch 1, dadurch gekennzeichnet, dass das Trägermaterial vorerwärmt ist.

12. Verfahren gemäss Anspruch 1, dadurch gekennzeichnet, dass es sich bei den Verbindungen der Formel I um (Tetrathiotetracen)Cl_{0,5}, (Tetrathiotetracen)Br_{0,5,} (Tetrathiotetracen)I_{0,75},(Tetraselenotetracen)Cl_{0,5}, (Tetraselenotetracen)Br_{0,5}, (2-Fluor- oder 2,3-Difluortetrathio- oder -tetraselenotetracen)Cl_{0,5}, (2-Fluor- oder 2,3-Difluortetrathio- oder -tetraselenotenacen)Br_{0,5} und (Tetrathiotetracen)(CuCl₂)_{0,4 bis 0,6} handelt.

13. Verfahren gemäss Anspruch 1, dadurch gekennzeichnet, dass es sich bei den thermoplastischen Kunststoffen um Polyolefine, Polystyrol, Polyvinylchlorid, Polyvinylidenchlorid, Polyvinylidenfluorid, Polyacrylate, Polymethacrylate, Polyamide, Polyester, Polycarbonate, aromatische Polysulfone, aromatische Polyether, aromatische Polyethersulfone, Polyimide und Polyvinylcarbazol handelt.

14. Verfahren gemäss Anspruch 1, dadurch gekennzeichnet, dass die Temperatur der Lösungen im Verfahren a) 20 bis 350°C und im Verfahren b) von 50 bis 350 °C beträgt.

15. Verfahren gemäss Anspruch 1, dadurch gekennzeichnet, dass das Mengenverhältnis von Cl-, Br- oder I-haltiger organischer Verbindung zu den Verbindungen der Formel II von 10:1 bis 1:5 beträgt.

16. Verfahren gemäss Anspruch 1, dadurch gekennzeichnet, dass es sich bei den halogenierten organischen Verbindungen um Tetrabrommethan, Bromoform, Trichlorbrommethan, Hexachlorpropen, Hexachlorcyclopropan, Hexachlorcyclopentadien, Hexachlorethan, N-Chlorsuccinimid, Octachlorpropan, n-Octachlorbutan, n-Decachlorbutan, Tetrabromethan, Hexabromethan, Tetrabrom-o-benzochinon, 2,4,4,6-Tetrabrom-2,5-cyclohexadienon, Hexabrombenzol, Chloranil, Hexachloraceton, 1,4,5,6,7,7-Hexachlor-5-norbornen-2,3-dicarbonsäure, 1,2,5,6,9,10-Hexabromcyclododecan, Tetrachlorethylen, Perchlorcyclopentadien, Perchlorbutadien, Dichloracetaldehyd-diethylacetal, 1,4-Dichlor-2-buten, 1,3-Dichlor-2-buten, 3,4-Dichlor-1-buten, Tetrachlorcyclopropen, 1,3-Dichloraceton, 2,3,5,6-Hexachlor-p-xylol, 1,4-Bis(trichlormethyl)-benzol, 1,3-Dibrompropan, 1,6-Dibromhexan, 3-Chlorpropionsäureethylester, 3-Chlortoluol, 2-Chlorpropionsäuremethylester, 2-Chloracrylnitril, Trichloressigsäureethylester, 1,2,3-Trichlorpropan, 1,1,2-Trichlorethan, Chlorameisensäurebutylester, Trichlorethylen, 2,3-Dichlormaleinsäureanhydrid, 1,12-Dibromdodecan, α,α'-Dibrom-p-xylol, α,α'-Dichlor-o-xylol, Phenacylchlorid oder -bromid, 1,10-Dibromdecan, α,α'-Dichlor-p-xylol, α,α'-Dibrom-m-xylol, Jodacetonitril, 2,3-Dichlor-5,6-dicyanobenzochinon, 2,3-Dichlorpropionsäuremethylester, 1-Brom-2-chlorethan, 1-Brom-2-chlorpropan, Chlorameisensäure-2-bromethylester, Iodessigsäureethylester, N-Chlor-, N-Brom- oder N-Iodsuccinimid oder -phthalsäureimid, oder Mischungen hiervon handelt.

17. Verfahren gemäss Anspruch 1, dadurch gekennzeichnet, dass die organische Verbindung Tri(C₁-C₆-Alkyl)ammoniumhydrochlorid ist, und zusätzlich wässrige H₂O₂-Lösung mitverwendet wird.

18. Verfahren gemäss Anspruch 1, dadurch gekennzeichnet, dass das Lösungsmittel bei einer Temperatur von 50 bis 250 °C verdampft wird.

## Claims

1. A process for the production of an electrically conductive layer on a substrate, in which the electrically conductive layer consists of a network of crystal needles of electrically conductive radical cation salts of formula (I) 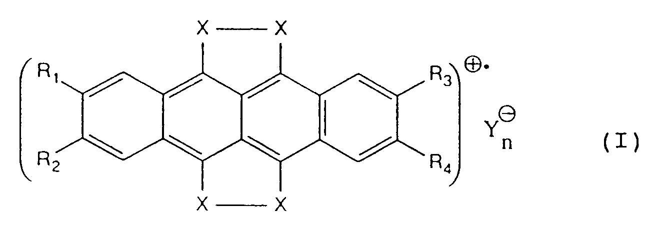 and the network may be embedded in a polymer matrix, wherein X is S, Se or Te, n is a number from 0.3 to 0.9, R₁ is H, F, Cl or CH₃, and R₂, R₃ and R₄ are H, or R₁, R₂, R₃ and R₄ are each F or CH₃, or R₁ and R₂ are each F, Cl or CH₃ and R₃ and R₄ are each H, or R₁ and R₃ or R₁ and R₄ are each F and R₂ and R₄ or R₂ and R₃ are each H, and Y is Cl, Br, I or CuCl₂, by coating at least one surface of the substrate with
a) a suspension of crystal needles of the radical cation salts of formula I in an inert solvent that may additionally contain, in dissolved form, a thermoplastic plastics material or at least one starting compound for a thermosetting plastics material, or
b) a solution of (b1) a compound of formula II 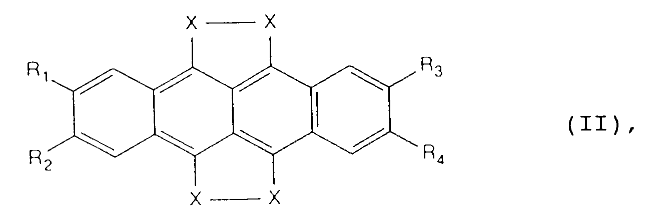 wherein R₁, R₂, R₃, R₄ and X are as previously defined, (b2) a monomeric, oligomeric or polymeric organic compound that contains chlorine, bromine or iodine and that forms with a compound of formula II, under the action of heat, a compound of formula I, or anhydrous CuCl₂, a CuCl₂ aquocomplex or a CuCl₂ solvent complex, and (b3), if desired, a thermoplastics plastics material or at least one starting compound for a thermosetting plastics material, in an inert solvent,
and subsequently evaporating the solvent, which process comprises applying the layer to the substrate by spraying through nozzles.

2. A process according to claim 1, wherein the diameter of the nozzle orifices is from 0.1 to 10 mm.

3. A process according to claim 1, wherein the pressure for the spraying is produced by a propellant gas.

4. A process according to claim 3, wherein argon or nitrogen is used as the propellant gas.

5. A process according to claim 3, wherein the pressure of the propellant gas is from 10⁴ to 10⁶ Pa.

6. A process according to claim 1, wherein the temperature of the nozzle head is in the range from room temperature to 250°C.

7. A process according to claim 1, wherein the distance of the nozzle heads from the substrate is from 1 to 100 cm.

8. A process according to claim 1, wherein one nozzle head or more than one nozzle head simultaneously is/are used.

9. A process according to claim 1, whrein the concentration of the compounds of formulae I and II is from 0.1 % by weight to 20 % by weight, based on the suspension or solution.

10. A process according to claim 1, wherein the concentration of the thermoplastic plastics material or starting compound(s) for thermosetting plastics material(s) in the solution is up to 60 % by weight.

11. A process according to claim 1, wherein the substrate is preheated.

12. A process according to claim 1, wherein the compound of formula I is selected from the group consisting of (tetrathiotetracene)Cl_{0.5}, (tetrathiotetracene)Br_{0.5}, (tetrathiotetracene)I_{0.75}, (tetraselenotetracene)Cl_{0.5}, (tetraselenotetracene)Br_{0.5}, (2-fluoro- or 2,3-difluorotetrathio- or -tetraselenotetracene)Cl_{0.5}, (2-fluoro- or 2,3-difluorotetrathio- or -tetraselenotetracene)Br_{0.5} and (tetrathiotetracene)(CuCl₂)_{0.4 to 0.6}.

13. A process according to claim 1, wherein the thermoplastics plastic material is selected from the group consisting of polyolefins, polystyrene, polyvinyl chloride, polyvinylidene chloride, polyvinylidene fluoride, polyacrylates, polymethacrylates, polyamides, polyesters, polycarbonates, aromatic polysulfones, aromatic polyethers, aromatic polyether sulfones, polyimides and polyvinylcarbazole.

14. A process according to claim 1, wherein the temperature of the solution in process a) is in the range from 20 to 350°C and the temperature of the solution in process b) is in the range from 50 to 350°C.

15. A process according to claim 1, wherein the ratio of the organic compound containing chlorine, bromine or iodine to the compound of formula II is from 10:1 to 1:5.

16. A process according to claim 1, wherein the halogenated organic compound is selected from the group consisting of tetrabromomethane, bromoform, trichlorobromomethane, hexachloropropene, hexachlorocyclopropane, hexachlorocyclopentadiene, hexachloroethane, N-chlorosuccinimide, octachloropropane, n-octachlorobutane, n-decachlorobutane, tetrabromoethane, hexabromoethane, tetrabromo-o-benzoquinone, 2,4,4,6-tetrabromo-2,5-cyclohexadienone, hexabromobenzene, chloranil, hexachloroacetone, 1,4,5,6,7,7-hexachloro-5-norbornene-2,3-dicarboxylic acid, 1,2,5,6,9,10-hexabromocyclododecane, tetrachloroethylene, perchlorocyclopentadiene, perchlorobutadiene, dichloroacetaldehyde diethyl acetal, 1,4-dichlorobut-2-ene, 1,3-dichlorobut-2-ene, 3,4-dichlorobut-1-ene, tetrachlorocyclopropene, 1,3-dichloroacetone, 2,3,5,6-hexachloro-p-xylene, 1,4-bis(trichloromethyl)benzene, 1,3-dibromopropane, 1,6-dibromohexane, ethyl 3-chloropropionate, 3-chlorotoluene, methyl 2-chloropropionate, 2-chloroacrylonitrile, ethyl trichloroacetate, 1,2,3-trichloropropane, 1,1,2-trichloroethane, butyl chloroformate, trichloroethylene, 2,3-dichloromaleic anhydride, 1,12-dibromododecane, α,α'-dibromo-p-xylene, α,α'-dichloro-o-xylene, phenacyl chloride and bromide, 1,10-dibromodecane, α,α'-dichloro-p-xylene, α,α'-dibromo-m-xylene, iodoacetonitrile, 2,3-dichloro-5,6-dicyanobenzoquinone, methyl 2,3-dichloropropionate, 1-bromo-2-chloroethane, 1-bromo-2-chloropropane, 2-bromoethyl chloroformate, ethyl iodoacetate, N-chloro-, N-bromo- and N-iodo-succinimide and -phthalimide, or mixtures thereof.

17. A process according to claim 1, wherein the organic compound is tri(C₁₋₆alkyl)ammonium hydrochloride, and aqueous H₂O₂ solution is additionally used concurrently.

18. A process according to claim 1, wherein the solvent is evaporated at a temperature in the range from 50 to 250°C.

## Revendications

1. Procédé de préparation d'une couche électroconductrice sur un matériau support, la couche électroconductrice est formée d'un réseau d'aiguilles cristallines de sels à cations radicalaires électroconducteurs de formule (I) 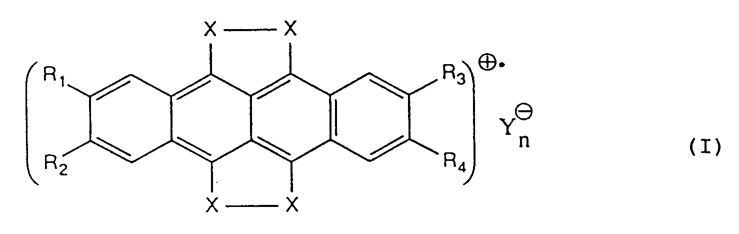 et le réseau peut être noyé dans une matrice de matière polymère, dans laquelle X désigne S, Se ou Te, n un nombre de 0,3 à 0,9, R₁ désigne H, F, Cl ou CH₃ et R₂, R₃ et R₄ H, ou bien R₁, R₂, R₃ et R₄ représentent chacun F ou CH₃, ou R₁ et R₂ chacun F, Cl ou CH₃ et R₃ et R₄ chacun H, ou R₁ et R₃ ou R₁ et R₄ signifie chacun F et R₂ et R₄ ou R₂ et R₃ chacun H, et Y désigne Cl, Br, I ou CuCl₂, par enduction d'au moins une surface du support avec a) une suspension des aiguilles cristallines des sels à cations radicalaires de formule I dans un solvant inerte pouvant également contenir en solution une matière thermoplastique ou au moins un précurseur de matières duroplastiques, ou bien b) une solution de (b1) un composé de formule II 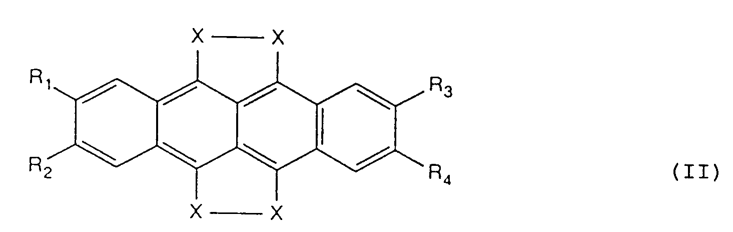 dans laquelle R₁, R₂, R₃, R₄ et X ont Les signification donnée peécédemment, (b2) un composé organique monomère, oligomère ou polymère et contenant du chlore, du brome ou de l'iode, formant avec un composé de formule II sous l'action de la chaleur un composé de formule I, ou bien du CuCl₂ anhydre, un aquocomplexe de CuCl₂ ou un complexe de CuCl₂ et d'un solvant, et (b3) le cas échéant une matière thermoplastique ou au moins un précurseur d'une de matières duroplastiques, dans un solvant inerte, puis évaporation du solvant, procédé caractérisé en ce que l'on dispose la couche sur le support par pulvérisation par des buses.

2. Procédé selon la revendication 1, caractérisé en ce que les ouvertures des buses ont un diamètre de 0,1 à 10 mm.

3. Procédé selon la revendication 1, caractérisé en ce que l'on crée la pression de pulvérisation par un gaz propulseur.

4. Procédé selon la revendication 3, caractérisé en ce que le gaz propulseur est de l'argon ou de l'azote.

5. Procédé selon la revendication 3, caractérisé en ce que la pression du gaz propulseur est de 10⁴ à 10⁶ Pa.

6. Procédé selon la revendication 1, caractérisé en ce que la température de la tête des buses est comprise entre la température ambiante et 250 °C.

7. Procédé selon la revendication 1, caractérisé en ce que la distance entre les têtes de buses et le support est de 1 à 100 cm.

8. Procédé selon la revendication 1, caractérisé en ce que l'on opère avec une seule tête de buse ou avec plusieurs à la fois.

9. Procédé selon la revendication 1, caractérisé en ce que la concentration des composés de formules I et II est de 0,1 à 20 % en poids, par rapport respectivement à la suspension ou à la solution.

10. Procédé selon la revendication 1, caractérisé en ce que la concentration de la solution en matières thermoplastiques ou en précurseurs de matières duroplastiques s'élève jusqu'à 60 % en poids.

11. Procédé selon la revendication 1, caractérisé en ce que l'on chauffe au préalable le matériau support.

12. Procédé selon la revendication 1, caractérisé en ce que, pour les composés de formule I, il s'agit de (tétrathio-tétracène)Cl_{0,5}, (tétrathio-tétracène)Br_{0,5}, (tétrathio-tétracène)I_{0,75}, (tétraséléno-tétracène Cl_{0,5}, (tétraséléno-tétracène)Br_{0,5}, (2-fluoro- ou 2,3-difluoro-tétrathio- ou -tétraséléno-tétracène)Cl_{0,5}, (2-fluoro- ou 2,3-difluoro-tétrathio- ou -tétrasélénotétracène)Br_{0,5} et (tétrathio-tétracène)(CuCl₂)_{0,4 à 0,6}.

13. Procédé selon la revendication 1, caractérisé en ce qu'il s'agit, pour les matières thermoplastiques, de polyoléfines, polystyrène, polychlorure de vinyle, polychlorure de vinvlidène, polyfluorure de vinylidène, polyacrylates, polyméthacrylates, polyamides, polyesters, polycarbonates, polysulfones aromatiques, polyéthers aromatiques, polyéther-sulfones aromatiques, polyimides et polyvinylcarbazole.

14. Procédé selon la revendication 1, caractérisé en ce que la température des solutions est de 20 à 350 °C dans le procédé a), et de 50 à 350 °C dans le procédé b).

15. Procédé selon la revendication 1, caractérisé en ce que le rapport en quantités du composé organique contenant du chlore, du brome ou ou de l'iode aux composés de formule II est compris entre 10:1 et 1:5.

16. Procédé selon la revendication 1, caractérisé en ce que, pour les composés organiques halogénés, il s'agit de tétrabromométhane, bromoforme, trichlorobromométhane, hexachloropropène, hexachlorocyclopropane, hexachlorocyclopentadiène, hexachloréthane, N-chlorosuccinimide, octachloropropane, n-octachlorobutane, n-décachlorobutane, tétrabromométhane, hexabromométhane, tétrabromo-o-benzoquinone, 2,4,4,6-tétrabromo-2,5-cyclohexadiénone, hexabromobenzène, chloranile, hexachloracétone, acide 1,4,5,6,7,7-hexachloro-5-norbornène-2,3-dicarboxylique, 1,2,5,6,9,10-hexabromocyclododécane, tétrachloréthylène, perchlorocyclopentadiène, perchlorobutadiène, dichloracétaldéhydediéthylacétal, 1,4-dichloro-2-butène, 1,3-dichloro-2-butène, 3,4-dichloro-1-butène, tétrachlorocyclopropène, 1,3-dichloracétone, 2,3,5,6-hexachloro-p-xylène, 1,4-bis-(trichlorométhyl)-benzène, 1,3-dibromopropane, 1,6-dibromohexane, 3-chloropropionate d'éthyle, 3-chlorotoluène, 2-chloropropionate de méthyle, 2-chloracrylonitrile, trichloracétate d'éthyle, 1,2,3-trichloropropane, 1,1,2-trichloréthane, chloroformiate de butyle, trichloréthylène, anhydride de l'acide 2,3-dichloromaléique, 1,12-dibromododécane, α,α'-dibromo-p-xylène, α,α'-dichloro-o-xylène, chlorure ou bromure de phénacyle, 1,10-dibromodécane, α,α'-dichloro-p-xylène, α,α'-dibromo-m-xylène, iodoacétonitrile, 2,3-dichloro-5,6-dicyanobenzoquinone, 2,3-dichloropropionate de méthyle, 1-bromo-2-chloréthane, 1-bromo-2-chloropropane, chloroformiate de 2-brométhyle, iodoacétate d'éthyle, N-chloro-, N-bromo- ou N-iodo-succinimide ou -phtalimide, et leurs mélanges.

17. Procédé selon la revendication 1, caractérisé en ce que le composé organique est un chlorhydrate de tri(alkyl en C₁-C₆)-ammonium et est de plus employé conjointement avec une solution aqueuse de H₂O₂.

18. Procédé selon la revendication 1, caractérisé en ce que l'on évapore le solvant à une température de 50 à 250 °C.
